(19) Europäisches Patentamt
European Patent Office
Office européen des brevets

(11) EP 0 824 920 A1

(12) **EUROPEAN PATENT APPLICATION**
published in accordance with Art. 158(3) EPC

(43) Date of publication:
25.02.1998 Bulletin 1998/09

(51) Int. Cl.$^6$: **A61L 27/00**, A61K 6/08,
A61C 5/08

(21) Application number: 96913712.4

(22) Date of filing: 09.05.1996

(86) International application number:
PCT/JP96/01230

(87) International publication number:
WO 96/35461 (14.11.1996 Gazette 1996/50)

(84) Designated Contracting States:
DE FR GB

(30) Priority: 10.05.1995 JP 135707/95
10.05.1995 JP 135708/95

(71) Applicant:
KABUSHIKI KAISYA ADVANCE
Chuo-ku Tokyo 103 (JP)

(72) Inventor: SHIN, Yoshiharu
Higashimurayama-shi, Tokyo 189 (JP)

(74) Representative:
Rinuy, Santarelli
14, avenue de la Grande Armée
75017 Paris (FR)

(54) **BLOOD VESSEL SUBSTITUTE, TOOTH CROWN SUBSTITUTE, AND PROCESS FOR PRODUCING THE SAME**

(57) A blood vessel substitute comprising a tubular body of a mixture containing an ultrafine powder of hydroxyapatite and an elastomer or a crown substitute comprising a mixture containing hydroxyapatite and a resin.

Fig.1

## Description

### TECHNICAL FIELD

The present invention relates to a blood vessel substitute, a crown substitute, and processes for the production of the same.

### BACKGROUND ART

In the past, as a blood vessel substitute in vein bypass or replacement surgery, patient's own vein or a porous blood vessel substitute using polymer materials including polyesters such as Dacron® polytetrafluoroethylenes such as Teflon® had been mainly used. Further, blood vessel substitutes on the inside surface of which had been affixed anti-blood coagulating enzymes such as urokinase, plasmin, plasmin substances dissolving blood clots had been used. When the enzymes or the plasmin substances lose their activity, however, the anti-clotting activity may be impaired. It is considered that there is not much sustainability of the activity after implantation in the body. Further, since the material is porous, blood easily leaks out from it, and therefore, it is necessary to immerse the material in blood in advance for preclotting to prevent leakage of blood. Even so, in major arterial systems where the blood pressure is high, the blood will leak out, so not only does the surgery become that much more complicated, but also any leaked blood accumulating in the body will become a factor causing new inflammation. To prevent leakage of the blood, the main practice at the present time is to treat the material with gelatin or collagen, but this often leads to a high fever after the surgery due to the antigen-antibody reaction to the foreign protein, and therefore, there are many clinical problems. As opposed to this, there are blood vessel substitutes which are not porous, such as blood vessel substitutes segmenting the inner surface of a polyurethane polymer material, but not only is it not possible to expect much in terms of the effect of prevention of clotting due to the segmentation, but also polyurethane itself lacks much biocompatibility, and therefore, generally these are not used much at all.

A blood vessel substitute must have each of the three features of (1) anti-clotting activity, that is, not causing the clotting of the blood, (2) bioaffinity with the blood vessel tissue and cells, and (3) compliance or viscoelasticity so as not to obstruct the flow of blood. The blood vessel substitutes used at the present time almost all give just anti-clotting activity. There is not necessarily sufficient bioaffinity in the material itself. On top of this, no blood vessel substitute having a compliance equal to that of natural blood vessels has yet been developed.

On the other hand, crown materials have to be used under harsh conditions of constant exposure to saliva and repeated biting, so have to be superior in load resistance, impact resistance, and other mechanical strength. Further, they are required to be harmless to the body, that is, superior in biocompatibility, and have to be aesthetic in appearance as compared with the natural teeth. Crown substitute materials have to meet all of the three requirements of mechanical strength, biocompatibility, and aesthetics and are placed under the harshest conditions among all biomaterials. Recently, further, machining systems using CAD/CAM and other computer control have been proposed for fabricating the crowns which is currently dependent on the skills of dental technicians. In such a case, the materials have to be able to be cut at dental technician centers. Further, the material must be one which is easy to color, part of the final finishing work for aesthetic purposes, and allows other conventional techniques of dental technicians to be put to use.

The crown substitute materials used at present are metals, resins, ceramics, porcelain, and various other artificial materials. Metals, however, are deficient in terms of aesthetics and colorability, resins are deficient in load resistance, and ceramics are deficient in impact resistance and cuttability and in terms of giving damage to the facing teeth. No ideal crown substitute material satisfying all of the requirements of mechanical strength, biocompatibility, aestheticness, cuttability, and colorability has yet been developed.

### DISCLOSURE OF INVENTION

Therefore, the object of the present invention is to provide a blood vessel substitute material which performs the function of a blood vessel substitute in the body over a long period of time, has anti-clotting activity, bioaffinity, and viscoelasticity, and therefore, is superior in function and practicality by mixing an ultrafine powder of hydroxyapatite with an elastomer.

Another object of the present invention is to provide a crown substitute material which performs the function of a crown in the oral cavity over a long period of time, has load resistance, impact resistance, bioaffinity, aethestics, cuttability, colorability, and reactivity with saliva by mixing hydroxyapatite. The reactivity with saliva not only gives a similar biological significance as natural teeth to the crown substitute, but also has the effect of naturally causing an aestheticness equal to that of the patient's own natural teeth through the adsorption action.

In accordance with the present invention, there is provided a blood vessel substitute comprising a tubular body of a mixture containing an ultrafine powder of hydroxyapatite and an elastomer.

In accordance with the present invention, there is also provided a process for producing a blood vessel substitute comprising mixing an ultrafine powder of hydroxyapatite with an elastomer, follwed by shaping the mixture

In accordance with the present invention, there is further provided a crown substitute comprising a mixture containing hydroxyapatite and a resin.

In accordance with the present invention, there is still further provided a process for producing a crown substitute comprising mixing an ultrafine powder of hydroxyapatite with a resin, followed by shaping and curing the same.

BRIEF DESCRIPTION OF DRAWINGS

The present invention will be explained in further detail below with reference to the attached drawings:

Fig. 1 to Fig. 3 are graphs of the relationship of the flexural strength (Fig. 1) of a composite material of a polymethylmethacrylate (PMMA) resin and hydroxyapatite (HAp) obtained in the later explained Example 2-3, its modulus (Fig. 2), and its compressive strength (Fig. 3) with the amount of HAp used; and

Fig. 4 is a view of the test pieces (dimensions in mm)used in the above measurements.

BEST MODE FOR CARRYING OUT THE INVENTION

Hydroxyapatite is known to be superior in affinity to the body and is being clinically used for artificial bone, percutaneous terminals, etc. Further, hydroxyapatite is known to strongly adsorb and remove many blood clotting factors. Artificial blood vessels using hydroxyapatite ceramics are being studied as well. Since it has a much higher hardness compared with the soft body tissue and lacks elasticity and flexibility, it is totally unsuitable for use for blood vessel substitutes as it would inflict damage to the inner wells of the blood vessel, and therefore, lacks practicality.

By homogeneously mixing an ultrafine powder of hydroxyapatite into an elastomer to make a composite material, however, it becomes possible to provide a blood vessel substitute material which performs the function of a blood vessel substitute in the body over a long period of time, has anti-clotting activity, bioaffinity, and viscoelasticity, and therefore, is superior in function and practicality. The elastomer has viscoelasticity and is rich in elastic deformability, so when the particles are mixed, the particles inevitably detach or escape. Therefore, they give phagocytosis by phagocytes in the body and can cause an inflammatory reaction. On the other hand, hydroxyapatite micropowders are extremely small in size and are not only easily taken into the phagocytes, but also easily dissolve under the acidic environment in the phagocytes and have an action of neutralizing the acidic environment in the surrounding tissue, so even if hydroxyapatite microparticles detach or escape, they by nature enable recovery from the inflammation in an extremely short time.

Further, it is reported that hydroxyapatite reacts with saliva and has an excellent ability to adsorb saliva protein. It has been learned that the inorganic component hydroxyapatite constituting the enamel of natural teeth adsorbs saliva protein and forms a thin film called a "pericle" on the surface which covers and protects the teeth. Further, hydroxyapatite dissolves a bit and maintains the oral cavity weakly alkaline to suppress the proliferation of s. mutans and other oral cavity bacteria and thereby contribute to maintenance of the health of the oral cavity. Further, it is known that organic compounds having a carboxyl group react well with hydroxyapatite. The pretreatment by a carboxylic acid or polycarboxylic acid covers the surface of the hydroxyapatite with an organic layer, which improves the wettability of the hydroxyapatite and resin.

The composition, shape and structure, mode of use, etc. of the blood vessel substitute material according to the present invention will be now explained in detail.

The "hydroxyapatite" in the present invention is not only the pure product of the chemical composition $Ca_{10}(PO_4)_6(OH)_2$, but also one containing 1 to 10% carbonic acid ($CO_3$) ions or fluorine ions or chlorine ions, in place of the OH ions. Further, to improve the strength, microporosity, solubility, and other properties, forms including Mg, Na, K, Al, Si, Fe, Mn, Zn, C, Sr, Pb, Ba, Ti, Zr, and other ions are also covered. Further, mixtures comprising these as main ingredients but improved in sinterability, strength, porosity, etc. by the addition and intermixture of $Ca(PO_4)_2$, $MgO$, $Na_2O$, $K_2O$, $CaF_2$, $Al_2O_3$, $SiO_2$, $CaO$, $Fe_2O_3$, $MnO_2$, $ZnO$, $C$, $SrO$, $PbO$, $BaO$, $TiO_2$, $ZrO_2$, and other known additives are also covered. Further, barium sulfate, tungsten oxide, and the like may be mixed for enhancing the X-ray image-formability. A hydroxyapatite having the above components means one with a Ca/P molar ratio of 1.66, but calcium deficient apatite, tricalcium phosphate, tetracalcium phosphate, hexacalcium phosphate, and other calcium phosphate compounds alone or in any composites of two or more of the same have functions substantially identical to hydroxyapatite.

In the case of a blood vessel substitute, ultrafine powder of hydroxyapatite of a particle size of not more than 5 μm, preferably 0.1 to 1 μm or so, is sintered at a temperature of less than 800°C, preferably 400°C, mixed with an elastomer compound, kneaded, then shaped.

As the above-mentioned elastomer, for example, one may be selected from thermoplastic elastomers such as a silicone rubber, butyl rubber, fluororubber, polyurethane, polystyrene elastomers such as isoprene rubber, styrene-butadiene rubber, or polyolefin elastomers such as hexine rubber, polyester elastomer, etc. To keep the reduction in strength of the matrix of the elastomer to a minimum, tubular body having a double layer or multilayer structure may be used, where the mixture of the hydroxyapatite and the elastomer is formed only on the innermost layer and/or outermost layer contacting the blood or the blood vessel tissue.

A- the resin which is mixed with the hydroxyapatite in the crown substitute according to the present invention, for example, one may be selected from a methacrylic acid ester resin or acrylic acid ester resin, a polycarbonate resin, epoxy resin, polysulfone resin, fluorine resin, etc. These resins may further include C, SiC, $SiO_2$, $Al_2O_3$, $ZrO_2$, glass fibers, ceramic fibers, ceramic whiskers, $TiO_2$, TiN, Ti, W, Mo, stainless steel, and other reinforcing materials and other fillers. Further, the above-mentioned carboxylic acid and polycarboxylic acid are general names for organic compounds having carboxyl groups and include saturated aliphatic acids such as lactic acid, pyruvic acid, citric acid, malic acid, butyric acid, maleic acid, malonic acid, formic acid, acetic acid, propionic acid, unsaturated fatty acids such as acrylic acid, linoleic acid. Further, their salts or esters may be used. Further, they may be used mixed with a conventional silane coupling agent.

The hydroxyapatite used for the crown substitute preferably is an ultrafine powder having a particle size of not more than 5 μm, more preferably having a size of 0.1 μm to 1 μm. Note that the hydroxyapatite used for the crown substitute may be a dense material having a relative density of at least 75% or a pulverized material of a particle size of 1 to 500 μm or a fibrous granulate. Note that in the case of the ultrafine powder, the hydroxyapatite is preferably sintered at a temperature of less than 800°C and in the case of a granulate at a temperature of at least 800°C.

The amount of the hydroxyapatite used in the present invention is 5 to 30% by weight of the content of the mixture in the case of a blood vessel substitute and not more than 90% by weight of the total amount of the mixture, preferably 50 to 75% by weight, in the case of a crown substitute. Further, for an inlay or other dental prosthesis, 50% by weight is sufficient.

EXAMPLES

The present invention will now be explained in further with reference to the Examples, but the present invention is, of course, not limited to these examples.

Example 1-1

Wet synthesized ultrafine powder of hydroxyapatite having a particle size of not more than 5 μm was dried at 400°C for one day and night, then was mixed in amounts of 5, 15, and 30% by weight with an addition type silicone rubber compound. These were each fully mixed, then formed into sheets having a thickness of 2 mm. Secondary vulcanization was then performed. Each of these was then tested for physical properties according to JIS K6310 Physical Test Methods for Vulcanized Rubber. The tensile strengths were 86, 83, 58, and 35 kgf/cm² for 0.5, 15, and 30% by weight addition of hydroxyapatite or a straight decline. Conversely, the hardnesses decreased linearly from 69, 71, 75, and 80. The tear strengths were 14, 24, 26, and 21 kgf/cm respectively, showing that the addition of hydroxyapatite causes the tear strength to increase and that the largest value is near 15% by weight addition.

Example 1-2

Wet synthesized ultrafine powder of hydroxyapatite having a particle size of not more than 5 μm was dried at 400°C for one day and night, then was mixed in amounts of 5, 15, and 30% by weight with an addition type silicone rubber compound. These were each fully mixed, then formed into sheets having a thickness of 2 mm. Secondary vulcanization was then performed. 15 mm x 15 mm materials containing 0.5, 15, and 30% by weight of hydroxyapatite were embedded under the skin of dogs. After two weeks and one, three, and six months, these were excised along with the surrounding tissue, then pathological tissue specimens prepared and observed under an optical microscope. The fibrous coverings formed around the composite materials were thinner and tissue reaction slighter as a general trend in the case of intermixture of hydroxyapatite than in the case of the silicone rubber alone. The thicknesses of the coverings around the composite materials after being embedded for six months were approximately 365, 290, 25, and 150 μm in the case of 0, 5, 15, and 30% by weight of hydroxyapatite. The surrounding covering was thinnest with 15% by weight of hydroxyapatite.

Example 1-3

Wet synthesized ultrafine powder of hydroxyapatite having a particle size of not more than 5 μm was dried at 400°C for one day and night, then was mixed in an amount of 15% by weight with an addition type silicone rubber compound. This was fully mixed, then formed into a tube of an inner diameter of 3 mm, an outer diameter of 3.5 mm, and a thickness of 0.25 mm. Secondary vulcanization was then performed. The shaped tube was immersed in 6N hydrochloric acid for one day except for the cut ends, whereupon the hydroxyapatite dissolved from the surface due to the hydrochloric acid and what had been a milky white colored tube became semitransparent. On the other hand, the control use silicone rubber failed to show any change from before the immersion. From this, it was confirmed that the ultrafine powder of hydroxyapatite were homogeneously distributed and exposed on the surface of the material as well.

Example 1-4

Wet synthesized ultrafine powder of hydroxyapatite having a particle size of not more than 5 μm was dried at 400°C for one day and night, then was mixed in an amount of 15% by weight with an addition type silicone rubber compound. This was fully mixed, then formed into a tube shape of an inner diameter of 3 mm, an outer diameter of 3.5 mm, and a thickness of 0.25 mm. Secondary vulcanization was then performed. The carotid artery of a dog was tied off at two locations, then the middle portion cut off and the two ends of a 30 mm length of the formed tube were inosculated with the cut ends of the blood vessel in the lateral direction carefully so as not to tear the same, then the tube buried and the progress observed. As a result, the composite material tube remained sufficiently open even after four weeks, while the control silicone rubber became blocked. Further, the histological reaction of the blood vessel around the inosculated portions was excellent and no inflammatory cellular infiltration was observed, while with the control silicone rubber, some cellular infiltration was observed at the interface of the inosculated portions.

Example 1-5

A silicone rubber tube body having an outer diameter of 4.5 mm and an inner diameter of 2.5 mm with a three-layer structure with a mixed layer of 15% by weight of ultrafine powder of hydroxyapatite mixed with silicone rubber formed at a thickness of 0.1 mm at each of the innermost layer and the outermost layer was formed. The two ends of the tube were inosculated with the cut ends of the carotid artery of a dog in the lateral direction carefully so as not to tear the same, then the tube buried and the progress observed. The composite material tube having the three-layer structure was sufficiently open even after being buried for four weeks, while the control silicone rubber was blocked. The tissue reaction of the blood vessel around the inosculated parts was also good and almost no inflammatory cellular infiltration was observed.

Example 2-1

Wet synthesized ultrafine powder of hydroxyapatite having a particle size of not more than 5 μm (average 1 μm) was dried at 400°C for one day and night, then was mixed in an amount of 20% by weight with a polymethyl methacrylate resin and the resultant mixture was polymerized at 60°C for about 10 hours. The material was milky white in color and close to that of a tooth. The compressive strength and flexural strength of the material were measured, whereupon they were found to be 1,200 kg/cm$^2$ and 850 kg/cm$^2$. On the other hand, the values of a polymethylmethacrylate resin without hydroxyapatite added were 750 kg/cm$^2$ and 700 kg/cm$^2$, respectively, showing that there was an improvement in the mechanical strength. This was cut by a small sized automatic cutting device, whereupon it was found that there was less of the tackiness characteristic of resins and the cuttability was improved compared with just a polymethylmethacrylate resin. Further, this was cut into a crown shape and surface adjusted in hue and buffed, whereupon it was possible to create a feeling close to that of a tooth.

Example 2-2

Wet synthesized ultrafine powder of hydroxyapatite was sintered at 1,200°C for two hours, then pulverized to a particle size of 1 to 50 μm (average 15 μm). The resultant dense granulate was treated for 5 minutes with 10% propionic acid, then mixed in an amount of 30% by weight with a polymethylmethacrylate resin. Further, this was polymerized at 60°C for 10 hours. The resultant mixture was a somewhat transparent feeling milky white color and a feeling close to that of a tooth. The compressive strength and flexural strength of the material were measured, whereupon they were found to be 1,500 kg/cm$^2$ and 950 kg/cm$^2$ or a greatly improved mechanical strength. This was cut by a small sized automatic cutting device, whereupon it was found that there was less of the tackiness characteristic of a resin and the cuttability was improved compared with just a polymethylmethacrylate. This was further cut into a crown shape and buffed, then was attached to the top of a tooth root using a dental adhesive. The resultant crown substitute when wet with saliva was superior in aesthetic appearance.

Example 2-3

A composite material of polymethylmethacrylate (PMMA) and hydroxyapatite (HAp) was produced in the following way based on the explanation in the reference (Mitsuhiro Kirigakubo: Research on Multiphase Type Biomedical Materials Having Bone Compatibility - Report 1: Mechanical Properties of hydroxyapatite-Synthetic Polymer-Metal Multiphase Material, Japanese Journal of Oral Surgery, vol. 32, no. 9: 1541-1560, 1986).

First, PMMA resin (made by Asahi Kasei, Terepellet 60N spherical resin) was dried at 80°C for 24 hours. The PMMA resin was then flattened into a pancake shape as if making a pancake while applying weak heat in a kneader. HAp powder (particle size of 1 to 10 μm, average 2.7 μm) was added to this, provisional sintering performed (800°C), then the resultant mixture was dried at 120°C for 2 to 3 hours (preferably one day and night) to prepare three types of samples with 30, 50, and 70% by weight content.

Further, the composite material was kneaded at a temperature of less than 100°C for 90 minutes, taken out of the mixing vessel, and cooled and solidified. The cooled and solidified mass was pulverized in the above mixing device to obtain a granular substance having diameters of less than 3 mm. The granular substance was shaped under the following conditions:

Cylinder temperature: H3 to H1: 210°C to 240°C
Nozzle temperature: Temperature not causing the material to droop from the front end
Die temperature: 60°C (however, 70°C just for sample with 70% by weight HAp) (standard for PMMA catalog value of 50 to 80°C)

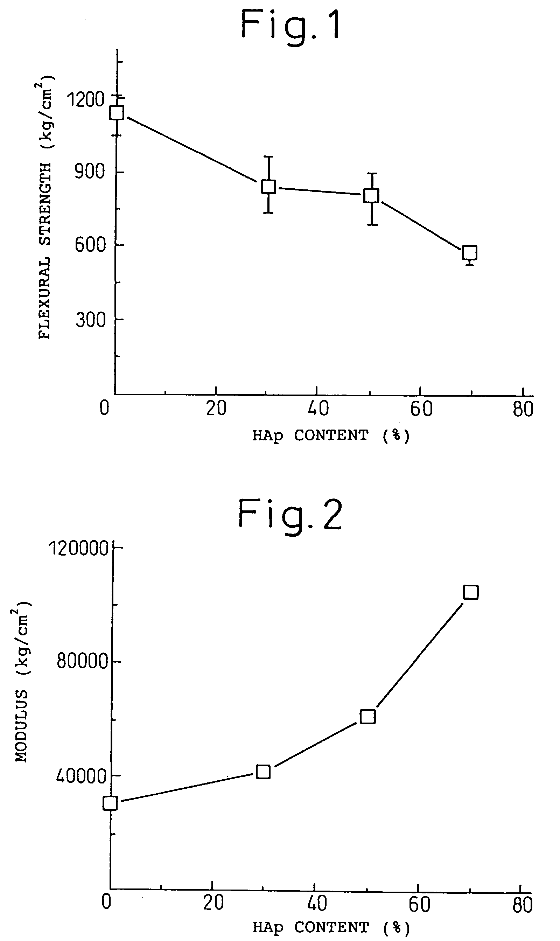

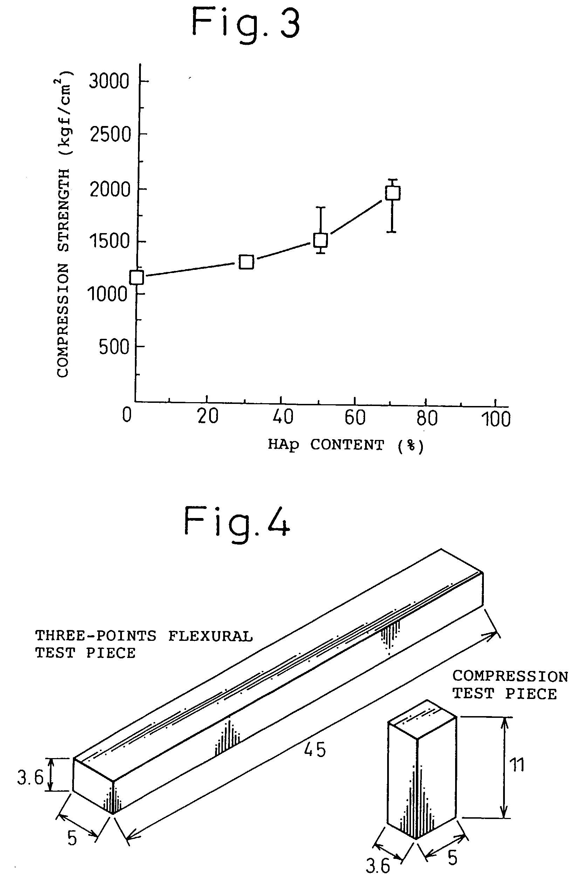

The PMMA + HAp composite material (test piece) shown in Fig. 4, shaped based on the above process of production, was measured for its flexural strength, modulus, and compressive strength. The results are shown in the graphs of Fig. 1 to Fig. 3.

Test Conditions

1. Three-Point Bending

- Model used: Autograph AG-2000A (made by Shimadzu Seisakusho)
- Span (distance between support points): 36 mm

  In JIS K7203, the length L between support points is defined as L-15h to 17h using the height h of the test piece. With the test piece used here, however, the ST ratio (distance between support points/height) was set to 10 as it was desired to use the injection molded article as it was (See Masahiro Kakeya et al.: Some Thoughts on Testing of Materials - Effects of Measurement Conditions on Flexural Strength -, Journal of the Japanese Society for Dental Materials and Devices, vol. 15, no. 1, pp. 29 to 37, 1996).

- Cross-head speed: 1.0 mm/min
  From JIS K7203,

$$V = Sr \times L^2/(6 h)$$

Where,

$Sr$: strain speed $(min^{-1}) = 0.01$
$L$: length between support points (mm)
$H$: height of test piece (mm)

$$V \geq 0.6$$

therefore, set to 1.0 mm/min.
- Test temperature: 23±1°C
- Pressing machine diameter: 2 mm
- Calculated data: yield load, maximum load, amount of deflection at time of maximum load

2. Measurement of Compressive Strength

- Height of test piece: 11 mm
  From JIS K7208,

$$h = k \times \lambda$$

$k$ (sectional secondary radius) $= \sqrt{(I/A)}$

Where,

$I$: sectional secondary mode
$A$: sectional area of test piece
$\lambda$: thickness/length ratio $\cdot \cdot$ in principle, 10

From which, h = 10.83, from which 11 mm is decided.
- Cross-head speed
  From JIS K7208,
  V = 0.3 h, from which 3.0 mm/min.
- Test temperature: 23±1°C
- Calculated data: yield point, maximum load

These were measured according to with the above conditions using basically the Hard Plastic Flexural Test Method K7203 and Plastic Compressive Test Method K7208.

From the above results, it was confirmed that the compressive strength is improved by increasing the amount of HAp added to the PMMA resin and that when over 50% a strength and ease of cutting sufficient for a dental prosthetic are obtained.

INDUSTRIAL APPLICABILITY

The blood vessel substitute of the present invention, in which 5 to 30% by weight of the ultrafine powder of hydroxyapatite is mixed in an elastomer, has the three requirements of anti-clotting activity, bioaffinity, and viscoelasticity and further is not porous, so does not require preclotting, and is extremely useful as an ideal blood vessel substitute - both for large blood vessels and small blood vessels. Further, by using a crown material obtained by mixing into ultrafine powder of a hard resin or dense granules of hydroxyapatite, it is possible to provide an ideal crown substitute superior in load resistance, impact resistance, bioaffinity, aestheticness, cuttability, colorability, and reactivity with saliva, and therefore, which is extremely useful.

**Claims**

1. A blood vessel substitute comprising of a tubular body of a mixture containing ultrafine powder of hydroxyapatite and an elastomer.

2. A blood vessel substitute as claimed in claim 1, wherein the ultrafine powder of hydroxyapatite is one sintered at a temperature of less than 800°C.

3. A blood vessel substitute as claimed in claim 1, wherein the content of the ultrafine powder of hydroxyapatite is 5 to 30% by weight of the total weight of the mixture.

4. A blood vessel substitute as claimed in claim 1, having at least a two-layer structure wherein a layer of the mixture of the elastomer and the ultrafine powder of hydroxyapatite is positioned at at least one of an inner surface of the tube and outer surface of the tube.

5. A process for producing a blood vessel substitute comprising mixing an elastomer with ultrafine powder of hydroxyapatite, followed by shaping the mixture.

6. A process for producing a blood vessel substitute as claimed in claim 5, wherein, before mixing with the elastomer, the ultrafine powder of hydroxyapatite is sintered at a temperature of less than 800°C.

7. A process for producing a blood vessel substitute as claimed in claim 5, wherein the content of the ultrafine powder of hydroxyapatite to be mixed with the elastomer is 5 to 30% by weight of the total weight of the mixture.

8. A process for producing a blood vessel substitute as claimed in claim 5, wherein the tubular body has at least a two-layer structure wherein a layer of the mixture of the elastomer and the ultrafine powder of hydroxyapatite is positioned at at least one of an inner surface of the tube and an outer surface of the tube.

9. A crown substitute comprising a mixture containing hydroxyapatite and a resin.

10. A crown substitute as claimed in claim 9, wherein the molar ratio (Ca/P) of the Ca and P of the hydroxyapatite mixed in the resin is 1.5 to 2.0.

11. A crown substitute as claimed in claim 9, wherein the hydroxyapatite mixed with the resin is ultrafine powder sintered at a temperature of less than 800°C.

12. A crown substitute as claimed in claim 9, wherein the hydroxyapatite mixed with the resin is a granulate sintered at a temperature of at least 800°C.

13. A crown substitute as claimed in claim 9, wherein the content of the hydroxyapatite mixed with the resin is not more than 75% by weight of the total amount of the mixture.

14. A crown substitute as claimed in claim 11, wherein the particle size of the ultrafine powder of μm. hydroxyapatite mixed with the resin is not more than 5

15. A crown substitute as claimed in claim 12, wherein the hydroxyapatite granulate mixed with the resin is a dense material having a relative density of at least 75%.

16. A crown substitute as claimed in claim 12, wherein the hydroxyapatite granulate mixed with the resin is a pulverized material having a particle size of 1 to 500 μm.

17. A crown substitute as claimed in claim 12, wherein the hydroxyapatite granulate mixed with the resin is a needle or fibrous material having a particle size of 1 to 500 μm.

18. A crown substitute as claimed in claim 9, wherein the hydroxyapatite is an ultrafine powder or granulate pretreated with a carboxylic acid, polycarboxylic acid, or a salt thereof.

19. A process for producing a crown substitute comprising mixing an ultrafine powder of hydroxyapatite with a-resin, followed by shaping and curing the same.

20. A process for producing a crown substitute as claimed in claim 19, wherein the molar ratio (Ca/P) of the Ca and P of the hydroxyapatite mixed with the resin is 1.5 to 2.0.

21. A process for producing a crown substitute as claimed in claim 19, wherein the hydroxyapatite mixed with the resin is an ultrafine powder sintered at a temperature of less than 800°C.

22. A process for producing a crown substitute as claimed in claim 19, wherein the hydroxyapatite mixed with the resin is a granulate sintered at a temperature of at least 800°C.

23. A process for producing a crown substitute as claimed in claim 19, wherein the content of the hydroxyapatite mixed with the resin is not more than 75% by weight of the total amount of the mixture.

24. A process for producing a crown substitute as claimed in claim 21, wherein the particle size of the ultrafine powder of hydroxyapatite mixed with the resin is not more than 5 μm.

25. A process for producing a crown substitute as claimed in claim 22, wherein the hydroxyapatite granulate mixed with the resin is a dense material having a relative density of at least 75%.

26. A process for producing a crown substitute as claimed in claim 22, wherein the hydroxyapatite granulate mixed with the resin is a pulverized material of a particle size of 1 to 500 μm.

27. A process for producing a crown substitute as claimed in claim 22, wherein the hydroxyapatite granulate mixed with the resin is a needle or fiber shape having a particle size of 1 to 500 μm.

28. A process for producing a crown substitute as claimed in claim 19, wherein the hydroxyapatite is an ultrafine powder or granulate pretreated with a carboxylic acid, polycarboxylic acid, or a salt thereof.

# Fig.1

# Fig.2

# Fig. 3

*X-axis:* HAp CONTENT (%)
*Y-axis:* COMPRESSION STRENGTH ($kgf/cm^2$)

# Fig. 4

THREE-POINTS FLEXURAL
TEST PIECE

COMPRESSION
TEST PIECE

# EP 0 824 920 A1

## INTERNATIONAL SEARCH REPORT

| International application No. |
| --- |
| PCT/JP96/01230 |

### A. CLASSIFICATION OF SUBJECT MATTER

Int. Cl$^6$   A61L27/00, A61K6/08, A61C5/08

According to International Patent Classification (IPC) or to both national classification and IPC

### B. FIELDS SEARCHED

Minimum documentation searched (classification system followed by classification symbols)

Int. Cl$^6$   A61L27/00, A61K6/08, A61C5/08

Documentation searched other than minimum documentation to the extent that such documents are included in the fields searched

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP, 61-135670, A (Mitsubishi Mining & Cement Co., Ltd.), June 23, 1986 (23. 06. 86) (Family: none) | 1 - 8 |
| X | JP, 3-82474, A (Junkosha K.K.), April 8, 1991 (08. 04. 91) (Family: none) | 1 - 8 |
| Y | JP, 60-259268, A (Advance K.K.), December 21, 1985 (21. 12. 85) (Family: none) | 1 - 8 |
| Y | JP, 63-242264, A (Nippon Steel Chemical Co., Ltd.), October 7, 1988 (07. 10. 88) (Family: none) | 1 - 8 |
| X | JP, 52-94309, A (Sterling Drug Inc.), August 8, 1977 (08. 08. 77) (Family: none) | 9 - 28 |
| X | JP, 58-121205, A (Sterling Drug Inc.), July 19, 1983 (19. 07. 83) (Family: none) | 9 - 28 |
| Y | JP, 59-62510, A (Rohm and Haas Co.), | 9 - 28 |

[X] Further documents are listed in the continuation of Box C.       [ ] See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| July 5, 1996 (05. 07. 96) | July 16, 1996 (16. 07. 96) |

| Name and mailing address of the ISA/ | Authorized officer |
| --- | --- |
| Japanese Patent Office | |
| Facsimile No. | Telephone No. |

Form PCT/ISA/210 (second sheet) (July 1992)

11

## INTERNATIONAL SEARCH REPORT

| International application No. |
| --- |
| PCT/JP96/01230 |

**C (Continuation). DOCUMENTS CONSIDERED TO BE RELEVANT**

| Category* | Citation of document, with indication, where appropriate, of the relevant passages | Relevant to claim No. |
| --- | --- | --- |
| | April 10, 1984 (10. 04. 84)<br>& US, 4433958, A & EP, 104000, A | |

Form PCT/ISA/210 (continuation of second sheet) (July 1992)